# EUROPEAN PATENT APPLICATION

(11) **EP 1 078 988 A1**
(43) Date of publication of application: **28.02.2001**
(21) Application number: 99306862.6
(22) Date of filing: 27.08.1999
(51) Int. Cl.: C12N 15/31, C07K 14/21, C12N 15/79, C12N 5/10, C07K 19/00, C12N 15/62, A61K 39/104, A61K 48/00

(54) **Pseudomonas fusion protein vaccines**

(71) Applicant: Academia Sinica, Nan-Kang, Taipei, 115 (TW)
(72) Inventor: Hwang, Jaulang, Nankang, Taipei (TW); Shang, Huey-Fang, Taipei (TW); Chen, Tzong-Yueh, Tainan (TW)
(74) Representative: Woods, Geoffrey Corlett

(57) **Abstract**

A polypeptide comprises segments:
(1) a receptor binding domain of a Pseudomonas exotoxin or functional variant thereof;
(2) a membrane translocation domain of a Pseudomonas exotoxin or functional variant thereof;
(3) a *Pseudomonas* lipoprotein I or functional variant thereof, or antigenic fragment of a *Pseudomonas* lipoprotein I or functional variant thereof; and
(4) an antigenic C-terminal fragment of a *Pseudomonas* porin protein F or functional variant thereof.

The polypeptide is useful in vaccine compositions.

## Description

*Pseudomonas aeruginosa* is a major cause of nosocomial infections in immunocompromised patients and in those suffering from cystic fibrosis, severe burns, or neoplasia (Bodey et al., Rev Infect Dis 5:279-311, 1983). In light of its resistance to antibiotics, infections caused by *P. aeruginosa* are especially difficult to treat (Hancock *et al.*, J. Antimicrob Chemother 18:653-656, 1986). Thus, there is a need for a safe and effective vaccine against this bacterium.

The invention is based on the discovery that a new fusion protein containing a *Pseudomonas* exotoxin A fragment and two *Pseudomonas* outer membrane protein fragments can elicit efficient protection against homologous and heterologous challenge in an animal model.

Accordingly the invention provides a polypeptide comprising segments:
(1) a receptor binding domain of a *Pseudomonas* exotoxin or functional variant thereof;
(2) a membrane translocation domain of a *Pseudomonas* exotoxin or functional variant thereof;
(3) a *Pseudomonas* lipoprotein I or functional variant thereof, or antigenic fragment of a *Pseudomonas* lipoprotein I or functional variant thereof; and
(4) an antigenic C-terminal fragment of a *Pseudomonas* porin protein F or functional variant thereof.

The C-terminal fragment or functional variant thereof (i.e., a contiguous piece of protein F that includes the C-terminal amino acid but excludes the N-terminal amino acid) can be at least 161 amino acids in length. The C-terminal fragment also can be less than 340 amino acids in length (e.g. less than 300, 250 or 200 amino acids in length). In addition, portions (1) and (2) can be adjacent in the polypeptide, such as when a *Pseudomonas* exotoxin fragment free of an ADP-ribosylation domain can be included as a single, contiguous portion in the polypeptide. Portions (1), (2) (3) and (4) singly or in combination can be derived from *Pseudomonas aeruginosa.*

The invention also includes a nucleic acid encoding a polypeptide of the invention, a vector containing the nucleic acid, and a cell (e.g., a eukaryotic cell) containing the nucleic acid. The nucleic acids, vectors, and cells can also be used as a vaccine and be introduced into an animal to produce the polypeptide of the invention.

This invention further includes a process for producing the polypeptide of the invention by incubating the cell containing the nucleic acid encoding the said polypeptide.

A receptor binding domain of a *Pseudomonas* exotoxin is an exotoxin fragment that is capable of specifically binding to a receptor for a *Pseudomonas* exotoxin. Likewise, a membrane translocation domain of a *Pseudomonas* exotoxin is an exotoxin fragment that facilitates membrane translocation in the context of a polypeptide or fusion protein of the invention.

An antigenic fragment, peptide, or protein is an amino acid sequence which includes an epitope specifically recognized by an antibody, T cell, or B cell.

A functional variant of one of the segments of a polypeptide of the invention is a polypeptide which is a variant of a wild-type amino acid sequence, the intended function of which in the fusion protein has not been completely abolished. A functional variant may be, for example, a fragment of a wild type sequence, a non-naturally occurring sequence or a fragment which has a non-naturally occurring sequence.

A functional variant preferably has at least 60% sequence identity to a wild type sequence, more preferably at least 70%, at least 80%, at least 90%, at least 95%, at least 97% or at least 99% sequence identity thereto over a region of at least 20, preferably at least 30, for instance at least 40, at least 60, at least 100 contiguous amino acids or over the full length of a wild type sequence.

The sequence of wild type segments can thus be modified to provide functional variants. Amino acid substitutions may be made, for example from 1, 2 or 3 to 10, 20 or 30 substitutions. The modified segment generally retains the function it is intended to contribute to the polypeptide of the invention. Conservative substitutions may be made, for example according to the following Table. Amino acids in the same block in the second column and preferably in the same line in the third column may be substituted for each other.

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

Functional variants of segments of a polypeptide of the invention may also be fragments of the wild type amino acid sequences. Such fragments typically retain the activity they are intended to have in the polypeptide of the invention.

Other preferred fragments include those which include an epitope. Suitable fragments will be at least 5, e.g. at least 10, at least 12, at least 15 or at least 20 amino acids in size. Epitope fragments may typically be up to 50, 60, 70, 80, 100, 150 or 200 amino acids in size. Polypeptide fragments of the segments may contain one or more (e.g. 1, 2, 3 or 5 to 10, 20 or 30) substitutions, deletions or insertions, including conservative substitutions. Epitopes may be determined by techniques such as peptide scanning techniques already known in the art. These fragments will be useful for obtaining antibodies to polypeptides of the invention.

Polypeptides of the invention may be in a substantially isolated form. It will be understood that the polypeptide may be mixed with carriers or diluents which will not interfere with the intended purpose of the polypeptide and still be regarded as substantially isolated. A polypeptide of the invention may also be in a substantially purified form, in which case it will generally comprise the polypeptide in a preparation in which more than 50%, e.g. more than 80%, 90%, 95% or 99%, by weight of the polypeptide in the preparation is a polypeptide of the invention.

Polypeptides of the invention may be chemically modified, e.g. post-translationally modified. For example, they may be glycosylated or comprise modified amino acid residues. They may also be modified by the addition of Histidine residues to assist their purification or by the addition of a signal sequence to promote their secretion from a cell. Such modified polypeptides and proteins fall within the scope of the term "polypeptide" of the invention.

Polypeptides of the invention may be modified for example by the addition of Histidine residues or a T7 tag to assist their identification or purification or by the addition of a signal sequence to promoter their secretion from a cell where the polypeptide does not naturally contain such a sequence.

Where a particular polypeptide or nucleic acid molecule is said to have a specific percent identity or conservation to a reference polypeptide or nucleic acid molecule, the percent identity or conservation is determined by the algorithm of Myers and Miller, CABIOS (1989), which is embodied in the ALIGN program (version 2.0), or its equivalent, using a gap length penalty of 12 and a gap penalty of 4 where such parameters are required. All other parameters are set to their default positions. Access to ALIGN is readily available. See, e.g., http://www2.igh.cnrs.fr/bin/align-guess.cgi on the Internet.

The polypeptides of the invention can be used to elicit antibodies and/or cell-mediated immunity against *Pseudomonas* bacteria in an animal (e.g., a mammal such as a human). As shown below, the polypeptides of the invention can further produce protective immunity in an animal challenged with *Pseudomonas* bacteria.

Other features or advantages of the present invention will be apparent from the following detailed description, and also from the claims.

The invention relates to a fusion protein that is suitable as a vaccine and contains at least three *Pseudomonas* antigens or antigenic fragments thereof. The fusion protein contains antigenic determinants present in the *Pseudomonas* exotoxin A protein, the Pseudomonas OprI gene product, and the *Pseudomonas* OprF gene product. A fusion protein containing these antigenic determinants was found to be superior to OprF or exotoxin A alone in rodent challenge assays *in vivo.*

Thus, the invention includes vaccine compositions (e.g., oral or injectable vaccines) containing at least one fusion polypeptide of the invention and optionally a pharmaceutically acceptable carrier, such as the diluents phosphate buffered saline or a bicarbonate solution (e.g., 0.24 M NaHCO₃). The carriers used in the composition are selected on the basis of the mode and route of administration, and standard pharmaceutical practice. Suitable pharmaceutical carriers and diluents, as well as pharmaceutical necessities for their use, are described in Remington's Pharmaceutical Sciences. An adjuvant, e.g., a cholera toxin, *Escherichia coli* heat-labile enterotoxin (LT), liposome, or immune-stimulating complex (ISCOM), can also be included in the vaccine composition.

The compositions can be formulated as a solution, suspension, suppository, tablet, granules, powder, capsules, ointment, or cream. In the preparation of these compositions, at least one pharmaceutical carrier is to be included. Examples of pharmaceutical carriers include solvent (e.g., water or physiological saline), solubilizing agent (e.g., ethanol, polysorbates, or Cremophor EL®), agent for making isotonicity, preservative, antioxidizing agent, excipient (e.g., lactose, starch, crystalline cellulose, mannitol, maltose, calcium hydrogen phosphate, light silicic acid anhydride, or calcium carbonate), binder (e.g., starch, polyvinylpyrrolidone, hydroxypropyl cellulose, ethyl cellulose, carboxy methyl cellulose, or gum arabic), lubricant (e.g., magnesium stearate, talc, or hardened oils), or stabilizer (e.g., lactose, mannitol, maltose, polysorbates, macrogols, or polyoxyethylene hardened castor oils) can be added. If necessary, glycerin, dimethylacetamide, 70% sodium lactate, a surfactant, or a basic substance such as sodium hydroxide, ethylenediamine, ethanolamine, sodium bicarbonate, arginine, meglumine, or trisaminomethane is added. Biodegradable polymers such as poly-D,L-lactide-co-glycolide or polyglycolide can be used as a bulk matrix if slow release of the composition is desired (see e.g., U.S. Patent Nos. 5,417,986, 4,675,381, and 4,450,150). Pharmaceutical preparations such as solutions, tablets, granules or capsules can be formed with these components. If the composition is administered orally, flavorings and colors can be added.

The compositions can be administered via any appropriate route, e.g., intravenously, intraarterially, topically, by injection, intraperitoneally, intrapleurally, orally, subcutaneously, intramuscularly, sublingually, intraepidermally, or rectally.

The amount of vaccine administered will depend, for example, on the particular vaccine antigen, whether an adjuvant is co-administered with the antigen, the type of adjuvant co-administered, the mode and frequency of administration, and the desired effect (e.g., protection or treatment), as can be determined by one skilled in the art. In general, the new vaccine antigens are administered in amounts ranging between 1 µg and 100 mg per adult human dose. If adjuvants are administered with the vaccines, amounts ranging between 1 ng and 1 mg per adult human dose can generally be used. Administration is repeated as necessary, as can be determined by one skilled in the art. For example, a priming dose can be followed by three booster doses at weekly intervals. A booster shot can be given at 8 to 12 weeks after the first immunization, and a second booster can be given at 16 to 20 weeks, using the same formulation. Sera or T-cells can be taken from the individual for testing the immune response elicited by the vaccine against *Pseudomonas* antigens. Methods of assaying antibodies or cytotoxic T-cells against a specific antigen are well known in the art. Additional boosters can be given as needed. By varying the amount of the fusion protein, the immunization protocol can be optimized for eliciting a maximal immune response.

The dose of the fusion polypeptide administered to a patient will depend generally upon the severity of the infection or condition, the age, weight, sex, and general health of the patient, and the potency of the fusion protein antigen.

Physicians, pharmacologists, and other skilled artisans are able to determine the most therapeutically effective treatment or prophylaxis regimen, which will vary from patient to patient.

Without further elaboration, it is believed that one skilled in the art can, based on the above disclosure and the description below, utilize the present invention to its fullest extent. The following set of Examples is to be construed as merely illustrative of how one skilled in the art can practice the invention and are not limitative of the remainder of the disclosure in any way. Any publications cited in this disclosure are hereby incorporated by reference.

### I. Production of Recombinant Fusion Protein

Expression plasmids coding for a *P. aeruginosa* exotoxin A (PE) having a deletion in the ADP-ribosylation domain; the OprF gene product; or a fusion protein (PEIF) containing the deleted exotoxin A, the OprP gene product, and the OprI gene product, were constructed as follows.

Plasmid pJH4, which encodes the full length PE (Hwang et al., Cell 48:129-136, 1987) was used as the starting material to construct various fusion protein expression plasmids. There are two AatII sites, one located at the boundary region of domain Ib and domain III of the PE gene, and the other located downstream of the PE gene near the EcoRI site beyond the 3' end. The second AatII site was destroyed by partially cleaving the plasmid pJH4 with AatII, followed by removing the single stranded end with mung bean nuclease and religation with T4 DNA ligase, thereby constructing plasmid pJMx. The AatII site immediately upstream of the domain III of PE was retained, while the other AatII site downstream of the PE gene was removed. The strategy used to construct the plasmid expressing PEIF was to cleave the PE gene with AatII and EcoRI, then replace domain III of PE with the Opr open reading frames. The expression vector pET-15b (Novagen), which contains the T7 promoter followed by a hexahistidine-tag sequence and polylinker, was used for expressing all the recombinant proteins in this study. A NdeI-PstI fragment containing the full length PE structural gene obtained from pJMx was subcloned into the corresponding restriction sites of pET-15b. The resulting plasmid, pET-15bJMx, encodes the full length PE with a N-terminal (His)₆-tag.

The OprI and OprF genes were amplified by PCR from the genomic DNA of *P. aeruginosa* strain PAOl. The oligonucleotides used for PCR were as follows. Primer I-1 (GCTGTTCTGGACGTCGGTTGCAGCAGCCAC; SEQ ID No:1) contains an AatII site (underlined) and corresponds to nucleotides 40-49 of the OprI coding sequence as described in Duchene et al., J Bacteriol 171:4130-4137, 1989. The reverse primer I-2 (CAGGTCGGAATTCCTATTACTTGCGGCT; SEQ ID NO:2) contains an EcoRI site (underlined) and corresponds to nucleotides 241-269 of the OprI coding sequence. The 229 bp PCR product resulting from a reaction using primers I-1 and I-2 was then used as the template for a second PCR using I-1 and I-3 (CGGAATTCCTATTACTCGAGGCTG; SEQ ID NO:3), which contains an XhoI site before the OprI stop codon and an EcoRI site. Both sites are underlined in the primer sequence. The 224 bp fragment produced from the second PCR was digested with AatII and EcoRI and subcloned in-frame into the AatII/EcoRI site of pET-15bJMx, thereby producing plasmid pET-15bJXx.

Oligonucleotide F-1 (GCAATGAACGCCCTCGAGCAGGGCCAG; SEQ ID NO:4) contains an XhoI site (underlined) and corresponds to nucleotides 55 to 81 of the OprF gene as described in Duchene et al., J Bacteriol 170:155-162, 1988, The reverse primer F-2 (TTTCTTTGAATTCTCAGCCGATTACTTGGC; SEQ ID NO:5) contains an EcoRI site (underlined) and corresponds to nucleotides 1045-1075 of the OprF open reading frame. The 1020 bp PCR product produced by using F-1 and F-2 was subcloned in-frame into the corresponding sites of pET-15bJIx to obtain plasmid pET-15bJIF, thereby producing a plasmid that expresses a fusion protein containing (His)₆-PE₁₋₄₀₅-OprI₁₉₋₈₃-OprF₂₄₋₃₅₀.

Other expression vectors were constructed as follows. Plasmid pET-15bF encoding (His)₆-OprF₂₄₋₃₅₀ was constructed by subcloning the XhoI-EcoRI PCR fragment of OprF into the corresponding sites in pET-15b. Plasmid pET-15bJJ10 encoding (His)₆-PE_{Δ398-613} was obtained by subcloning an NdeI to EcoRI fragment from pJJ10, which is described in Chow et al., J Biol Chem 264:18818-18823, 1989. The (His)₆-PE_{Δ398-613} protein is abbreviated PE(ΔIII). All plasmids were sequenced to ensure that no mutation was introduced by PCR.

For large-scale production of recombinant fusion proteins, the resulting plasmids were transformed into *E. coli* strain BL21(DE3). The bacteria containing expression plasmids as described above were cultured in LB broth at 37°C. When absorbance at 600 nm reached 0.3, isopropyl-1-thio-β-D-galactopyranoside was added to a final concentration of 0.5 mM, and the incubation was continued at 37°C for 90 minutes. Since all three His-tagged proteins were highly expressed in inclusion bodies, they could be purified from crude bacterial lysates using the Ni-charged His-Bind column (Novagen) under denaturing conditions. The purified proteins were analyzed by 12% SDS-PAGE and stained with Coomassie blue after electrophoresis as described in Hwang et al., J Biol Chem 264:2379-2384, 1989. The PE component of the expressed proteins, where present, were confirmed by immunoblotting with polyclonal rabbit anti-PE serum as described in Hwang et al., 1989. The OprI and OprF components of the fusion proteins, where expected to be present, were confirmed by immunoblotting with murine antisera against OprF and PEIF, which recognized the native OprF and OprI from the total cell lysates of six heterologous *P. aeruginosa* strains.

Thus, all three His-tagged recombinant proteins were efficiently expressed in bacteria and purified in one step using metal-affinity chromatography. Most of these proteins were purified to at least 90% homogeneity as analyzed by SDS-PAGE. Western blot analysis of these proteins indicated that PEIF, native PE, and PE(ΔIII), but not OprF, could be recognized by rabbit anti-PE polyclonal antibodies. The OprF and OprI portions of the fusion proteins were confirmed by Western blotting with murine or rabbit polyclonal anti-PEIP antisera, which recognized both native OprF (36 kD) and OprI (7 kD) from the total bacterial lysate of homologous (strain PAO1) and six heterologous *P. aeruginosa* strains (see below). No specific reactivity could be observed for an *E. coli* control lysate. A OprF-specific antiserum was able to specifically recognize the His-tagged OprF protein. Thus, properly folded fusion proteins as described above could be efficiently produced in large quantities.

### II. Immunization of Mice and Resulting Antibody Titers

Three groups of BALB/c mice (6 week old, 30 per group) were immunized with PEIF, OprF, and PE(ΔIII) absorbed in AlPO₄ adjuvant (2.5 mg/ml). The mice were immunized intraperitoneally on days 1, 21, and 35 at a dose of 0.5 µM, 0.75 µM, and 1 µM, respectively. Each does was a bolus of 0.2 ml.

Antisera to different recombinant proteins were generated in New Zealand white rabbits (two rabbits per group) by immunizing subcutaneously at six different locations near a lymph node. 200 µg of purified protein emulsified in Freund's complete adjuvant was administered on day 1, followed by 300 µg of emulsified protein on day 21 and 400 µg of emulsified protein on day 35.

The immune responses of the immunized mice or rabbits was monitored by ELISA (Dynatech Laboratories). The pooled antisera of each group were two-fold diluted (ranging from 1:50 to 1:12,800) with 0.5% BSA in PBS. The purified PE (ICN Biomedicals) and recombinant OprF were used to coat polyvinylchloride, flat-bottom, 96-well Falcon microtiter plates at a total protein concentration of 3 µg/ml. The antisera were also titered against whole bacterial cells of the six heterologous immunotype strains of P. aeruginosa (see below) . PBS washed bacteria (10⁴ cells in 0.1 ml of PBS) were coated on plates that had been pre-coated with poly-L-lysine (1 µg in 0.1 ml of PBS). The plates were then incubated overnight at 4°C. Peroxidase-conjugated antimouse or anti-rabbit immunoglobulin was used as the secondary antibody. The substrate solution contained 0.54 mg/ml 2,2'-azinobis(3-ethylbenzthiazoline-6-sulfonic acid) (ARTS) and 0.03% H₂O₂ in 0.1 M citric acid. A negative/positive cutoff value was defined as two standard deviations above the mean A₄₀₅ readings obtained from five non-immune rabbit or mice control antisera. The titer was defined as the reciprocal of the highest dilution of serum yielding a positive value. Some results were also expressed as optical density (OD₄₀₅) values obtained for a given serum dilution.

The immune responses of BALB/c mice after three doses of vaccine were determined by ELISA as described above on day 42. The PE(ΔIII) and PEIF proteins elicited antibodies specific for native PE. However, the antibody titers against PE in the PEIF-vaccinated antisera were higher than in the PE(ΔIII)-vaccinated antisera (1:3,200 vs. 1:800). On day 28 (7 days after the second vaccination), the average optical density of antisera against PE from PEIF-vaccinated groups at 1:500 dilution was 1.583 ± 0.095, while the average optical density of antisera against PE from PE(ΔIII)-vaccinated groups was 0.241 ± 0.072. The day 28 results were obtained after incubating the ELISA plate with ABTS for one hour at 25°C.

It is noted that both PEIF and PE(ΔIII) contained the same receptor binding and membrane translocation domains. The different antibody titers against native PE might be explained by the following. First, OprF might function as a T-helper lymphocyte carrier protein to increase the immunogenicity of PEIF against PE. Second, the PE-B cell epitopes on PEIF may be spatially presented in a similar manner to the epitopes on native PE, whereas the exposure of epitopes on PE(ΔIII) may be different than that on native PE. In any event, the immunogenicity of the PEIF fusion protein was unexpectedly higher than what was expected from PE(ΔIII) alone.

Both of the PEIF and OprF vaccinated mice had high titers (1:12,800) of antibodies against the purified OprF. The average optical density of anti-PEIF was 0.963 ± 0.012, and the average optical density of anti-OprF was 1.204 ± 0.078. These densities were determined on a 1:3,200 dilution of sera collected seven days after the second vaccination. These results indicated that the position of OprF at the C-terminus of PEIF did not reduce its immunogenicity against OprF.

In order to determine whether the anti-PEIF antibodies could recognize the surface-exposed antigens on the intact bacterium, various strains of *P. aeruginosa* were coated on ELISA plates. These strains included PAO1 (ATCC 15692; seragroup 5), PA103 (ATCC 29260), a serogroup 2 strain (ATCC 33349), a serogroup 6 strain (ATCC 33354), a serogroup 8 strain (ATCC 33355), and a serogroup 12 strain (ATCC 33359). The mean titer from triplicate readings for antisera elicited by PEIF or OprF against whole cells was 1:3,200 for the various strains. Compared to normal mice sera, the antisera from mice immunized with PE(AIII) did not react with the whole cells of various strains (OD₄₀₅ < 0.06 at 1:1000 dilution). On the other hand, PEIF and OprF elicited antibodies against whole cells at high levels. These levels were confirm for both the PAOl strain, from which the fusion proteins were produced, as well as for the heterologous strains. There was no significant difference between the whole-cell antibody titers produced by PEIF vaccination as compared to the titers produced by OprF vaccination.

Thus, a fusion protein of the invention could induce a vigorous antibody response to various antigenic components included in the fusion protein. In the case of PEIF, the fusion protein unexpectedly elicited higher levels of anti-PE antibodies than an immunogen including PE alone.

### III. PE Neutralization Capacity of Antibodies from Immunized Mice

The ability of various rabbit antisera to neutralize the cytotoxicity of PE was determined in NIH 3T3 cells as generally described in Cryz et al., Infect Immun 52:161-165, 1986 and Pollack et al., Infect Immun 14:942-947, 1976. NIH 3T3 cells were seeded in a 96-well tissue culture plate at a density of 10⁴ cells per well in 200 µl, 24 hours prior to the cytotoxicity assay. PE (1 µg/ml) was mixed with an equal volume of various dilutions of antisera for 30 minutes at 37°C. Fifty microliters per well of the mixture was added to the cultured cells. The final concentration of PE was 100 ng/ml, which was 10X the minimal dose required for greater than 95% cell death. After incubating for 72 hours, the cells were washed once with PBS and stained with 0.05% crystal violet in 20% ethanol for 10 minutes at room temperature. The crystal violet-stained cells were lysed with 100% methanol. A₅₉₅ was then determined using an automated microplate reader. Each diluted antisera was assayed in eight replicates. Toxin neutralization was calculated by the following formula: Percent Neutralization = (average absorbance of eight wells containing antiserum and toxin - average background of stained wells without cells)/(average absorbance of eight wells containing antiserum without toxin - average background of stained wells without cells). The neutralization titer was defined as the reciprocal of the highest dilution reducing cytotoxicity by 50%.

The antibodies elicited by PEIF and PE(ΔIII) were capable of neutralizing the cytotoxicity of PE on NIH3T3 cells. Both of the anti-PEIF and anti-PE(ΔIII) antisera (diluted 1:10) were able to completely inhibit the PE cytotoxicity. The cytotoxicity neutralization titers of antisera elicited by PEIP and PE(ΔIII) were 561 and 374, respectively. In general, the neutralizing capacity of the sera correlated well with the ELISA titers against PE.

### IV. Opsonization Capacity of Antibodies from Immunized Mice

The ability of PEIF vaccinated antisera to promote the uptake of *P. aeruginosa* by murine peritoneal macrophages was determined by the visual phagocytosis assay as generally described in Gilleland et al., Current Microbiol 24:1-7, 1992. The bacteria used in this assay were P. aeruginosa strains PAOl, PA103, and the serogroup 2 bacteria (see listing of bacteria above). Bacteria were grown to mid-log phase, washed twice in cold PBS and suspended in RPMI 1640 at a concentration of 10⁸ cfu/ml. Opsonization was performed by tumbling the bacteria at 37°C for 30 minutes in 10% (v/v) complement-inactivated murine antisera obtained from various vaccinated groups on day 42. Opsonized bacterial suspensions were then washed with PBS and resuspended in serum-free RPMI.

Macrophages were harvested from 6 to 8-week old BALB/c mice by intraperitoneal injection of 2 ml thioglycolate broth (Difco) 4 days before harvesting. Peritoneal exudate cells were collected by washing the peritoneal cavity with 5 ml of PBS. After washing with cold RPMI 1640, the pelleted macrophages were resuspended in RPMI 1640 containing 10% fetal calf serum at a concentration of 2 X 10⁵ cells/ml. Macrophages (1 ml) were added to individual wells of a 24-well plate containing 11 mm-diameter round glass coverslips. The cells were incubated on the coverslips for 2 hours at 37°C in a 5% CO₂ environment. After incubation, non-adherent cells were washed off, and the medium was replaced with 1 ml of pre-opsonized bacterial suspension (10⁷ cfu/ml) in serum-free RPMI 1640. Phagocytosis was allowed to proceed for 30 minutes at 37°C in a 5% CO₂ environment. After ingestion, extracellular bacteria were removed by washing the monolayer macrophages three times with cold PBS. Fixed coverslips were stained with 1% crystal violet. Intracellular bacteria within each of 100 cells were counted by oil immersion light microscopy. Phagocytosis of *P. aeruginosa* was expressed as the percentage of macrophages with less than 6, 6 to 19, or greater 19 bacteria ingested. All experiments were repeated at least two times with macrophages from different mice. The results are summarized in Table 1 below.

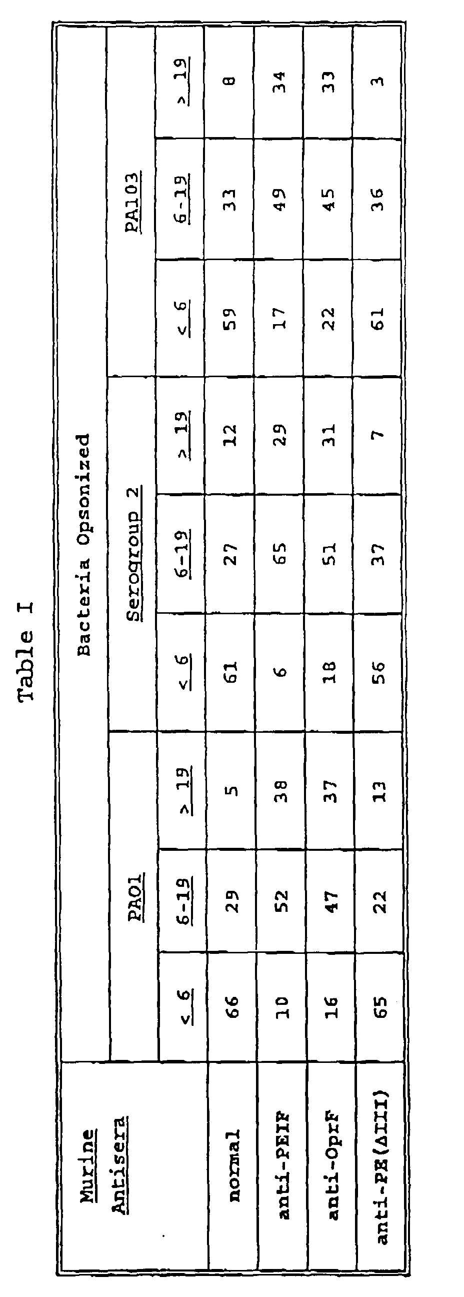

Few non-opsonized bacteria were phagocytosed by the macrophages (59% to 66%). Both the anti-PEIF and anti-OprF antisera led to a significant increase in opsonized uptake of heterologous (serogroup 2 and PA103) and homologous (PAOl) bacteria. In addition, as expected, anti-PE(ΔIII) sera did not opsonize bacteria since the target antigen is not a outer membrane protein. These results confirm the greater utility of the PEIF fusion protein in eliciting beneficial immune responses, including opsonization.

### V. Protection Against Bacterial Challenge

*P. aeruginosa* protection experiments were performed using the burned mouse model as generally described in Holder, Can J Microbiol 31:393-402, 1985. BALB/c mice were anesthetized with sodium pentobarbital (0.71 µg/g of body weight) and shaved dorsally prior to burning. A teflon template with a precisely cut window (35 X 25 mm) was firmly place over the shaved dorsal surface. A half milliliter of 95% ethanol (v/v) was pipetted onto the surface and ignited. Burns were precisely timed for 15 seconds. Approximately 30% of the total body surface were burned. Cold PBS (0.2 ml) containing to 5 X 10⁴ bacteria were immediately injected subcutaneously into the burned area. The challenge dose corresponded to about two times the LD₅₀ dose, which was calculated separately on non-vaccinated ethanol-burned mice for different bacteria. Significant protection was determined using Fisher's exact test using two-tailed p values. A result was significant if the *p* value was leas than 0.05.

The LD₅₀ values of *P. aeruginosa* strains PAOl, PA103, and the serogroup 2 bacteria were determined to be 5.36 X 10³, 3.87 X 10³, and 2.15 X 10⁴ cfu/burned mouse. Most *P. aeruginosa*-infected, burned, non-vaccinated mice died between 2 and 5 days after infection. Compared to the non-vaccinated control mice, the mice immunized with PELF or OprF were afforded significant protection against challenge with all bacteria (survival rate of 50-60%). The PE(ΔIII) protein was less effective in this model (survival rate of 20-30%), even though, as described above, this immunogen was capable of eliciting neutralizing antibodies against native PE. It was also observed that PEIF afforded significantly higher protection (80%) against challenge with PE-hyper-producing strain PA103 than OprF alone (40%). Thus, these results confirm that the PEIF fusion protein was a superior immunogen to the other immunogens examined, conferring protective immunity *in vivo.*

### Other Embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of this invention.

The plasmid of pET15bJIF have been deposited with the China Center for Type Culture Collection, (CCTCC, P. R. China) on 26 August 1999 in accordance with the Budapest Treaty, and assigned the accession No. CCTCC M 99015.

## Claims

1. A polypeptide comprising segments:
(1) a receptor binding domain of a *Pseudomonas* exotoxin or functional variant thereof;
(2) a membrane translocation domain of a *Pseudomonas* exotoxin or functional variant thereof;
(3) a *Pseudomonas* lipoprotein I or functional variant thereof, or antigenic fragment of a *Pseudomonas* lipoprotein I or functional variant thereof; and
(4) an antigenic C-terminal fragment of a *Pseudomonas* porin protein F or functional variant thereof.

2. The polypeptide of claim 1, wherein segments (1) and (2) are adjacent in the polypeptide.

3. The polypeptide of claim 1 or 2, wherein segments (1) and (2) are included in a *Pseudomonas* exotoxin fragment free of an ADP-ribosylation domain.

4. The polypeptide of any one of the preceding claims, wherein the segment (4) is 340 amino acids or shorter in length.

5. The polypeptide of claim 4, wherein the segment (4) is 300 amino acids or shorter in length.

6. The polypeptide of claim 5, wherein the segment (4) is 250 amino acids or shorter in length.

7. The polypeptide of claim 6, wherein the segment (4) is 200 amino acids or shorter in length.

8. The polypeptide of any one of the preceding claims, wherein segments (1), (2), (3) and (4) are derived from *Pseudomonas aeruginosa.*

9. The polypeptide of any one of the preceding claims, wherein segments (1), (2), (3) and (4) are presented in consecutive order from the N-terminus to the C-terminus of the polypeptide.

10. The polypeptide of any one of the preceding claims, wherein the C-terminal fragment of functional variant thereof is at least 161 amino acids in length.

11. The polypeptide of any one of the preceding claims, wherein the *Pseudomonas* lipoprotein I of functional variant thereof, or antigenic fragment of a *Pseudomonas* lipoprotein I of functional variant thereof is at least 64 amino acids in length.

12. A nucleic acid encoding a polypeptide of any one of the preceding claims.

13. A vector containing the nucleic acid of claim 12.

14. A cell containing the nucleic acid of claim 12 or vector of claim 13.

15. The cell of claim 14, wherein the cell is eukaryotic.

16. A vaccine composition comprising at least one polypeptide of any one of claims 1 to 11, and optionally a pharmaceutically acceptable carrier.

17. A vaccine composition comprising a nucleic acid of claim 12, a vector of claim 13 or a cell of claim 14 or 15 and optionally a pharmaceutically acceptable carrier.

18. The vaccine composition of claim 17, wherein the nucleic acid, vector or cell is introduced into an animal to produce a polypeptide of any one of claims 1 to 11.

19. The vaccine composition of any one of claims 16 to 18, for use in eliciting antibodies and/or cell-mediated immunity against *Pseudomonas* bacteria in an animal.

20. A process for producing a polypeptide of any one of claims 1 to 11 comprising:
a) the incubation of the cell of claim 14 or 15; and
b) the isolation and purification of the polypeptide.


### Annex to the application documents - subsequently filed sequences listing

SEQUENCE LISTING

<110> ACADEMIA SINICA

<120> PSEUDOMONAS FUSION PROTEIN VACCINES

<130> N77724 GCW

<140> 99306862.6
<141> 1999-08-27

<160> 5

<170> PatentIn Ver. 2.0

<210> 1
<211> 30
<212> DNA
<213> Artificial Sequence

<220>
<223> Description of Artificial Sequence: primer

<400> 1
gctgttctgg acgtcggttg cagcagccac 30

<210> 2
<211> 28
<212> DNA
<213> Artificial Sequence

<220>
<223> Description of Artificial Sequence: primer

<400> 2
caggtcggaa ttcctattac ttgcggct 28

<210> 3
<211> 24
<212> DNA
<213> Artificial Sequence

<220>
<223> Description of Artificial Sequence: primer

<400> 3
cggaattcct attactcgag gctg                                          24

<210> 4
<211> 27
<212> DNA
<213> Artificial Sequence

<220>
<223> Description of Artificial Sequence: primer

<400> 4
gcaatgaacg ccctcgagca gggccag                                       27

<210> 5
<211> 30
<212> DNA
<213> Artificial Sequence

<220>
<223> Description of Artificial Sequence: primer

<400> 5
tttctttgaa ttctcagccg attacttggc                                    30